# FASCICULE DE BREVET EUROPEEN

(11) **EP 3 223 647 B1**
(45) Date de publication et mention de la délivrance du brevet: **18.10.2023**
(21) Numéro de dépôt: 15801439.9
(22) Date de dépôt: 27.11.2015
(51) Int. Cl.: A45D 40/16, A61K 8/02, A61Q 1/06, A61Q 15/00

(54) **PROCÉDÉ DE FABRICATION D'UNE COMPOSITION COSMÉTIQUE**
VERFAHREN ZUR HERSTELLUNG EINER KOSMETISCHEN ZUSAMMENSETZUNG
PROCESS FOR MANUFACTURING A COSMETIC COMPOSITION

(30) Priorité: 28.11.2014 FR 1461680
(43) Date de publication de la demande: 04.10.2017
(73) Titulaire: L'OREAL, 75008 Paris (FR)
(72) Inventeur: LORENTE GONZALEZ, Sonia, 94300 Vincennes (FR); JAUNET, Clément, 78140 Velizy Villacoublay (FR)
(74) Mandataire: Lavoix
(86) Numéro de dépôt international: PCT/EP2015/077943
(87) Numéro de publication internationale: WO 2016/083585

(56) Documents cités:
- DE-A1- 3 327 771
- FR-A1- 2 976 777
- US-A- 5 160 689

## Description

La présente invention concerne un procédé de fabrication d'une composition cosmétique selon le préambule de la revendication 1.

La composition cosmétique contient au moins un produit cosmétique, notamment un produit de maquillage, un produit de soin, un produit de lavage ou un parfum, le produit cosmétique étant destiné à être appliqué sur une surface corporelle d'un utilisateur.

Plus généralement, par « produit cosmétique », on entend notamment au sens de la présente invention, un produit tel que défini dans le Règlement (CE) n°1223/2009 du Parlement Européen et du Conseil du 30 novembre 2009, relatif aux produit cosmétiques.

La composition cosmétique forme ainsi avantageusement un rouge à lèvres, un stick, une poudre hybride, un produit déodorant et/ou anti transpirant, un savon, un masque de visage, une cire de coiffage, ou/et un parfum solide.

Pour fabriquer un article comprenant une composition cosmétique, il est connu d'utiliser des procédés de mise en forme tels que le moulage, ou le coulage par exemple

De tels procédés impliquent de chauffer au moins un matériau cosmétique comprenant un composé fusible pour faire fondre le composé fusible, puis de couler le matériau cosmétique chauffé dans une cavité d'un moule.

Le matériau cosmétique est ensuite refroidi pour permettre la solidification du composé fusible, et une composition cosmétique structurée obtenue est extraite du moule.

Un tel procédé ne donne pas entière satisfaction, notamment en ce qui concerne la cadence de production maximale.

En particulier, lors du coulage du matériau chauffé, il est nécessaire de s'assurer que des bulles d'air ne sont pas créées, ce qui nuirait à l'esthétique du produit obtenu est à ses propriétés mécaniques. La vitesse de coulage doit donc être limitée.

Par ailleurs, pour accélérer la solidification du matériau cosmétique, il est possible de réduire la température des parois du moule. Ceci est peu utilisable en pratique, compte tenu des risques de condensation de l'humidité résiduelle dans la cavité de moulage, qui peut nuire également à la qualité de la composition obtenue.

Dans le cas de la coulée d'un bâton de produit cosmétique, il est par ailleurs nécessaire de monter le bâton sur son support après la fabrication du bâton, ce qui provoque dans certains cas des défauts de qualité, compte tenu des tolérances serrées entre le bâton et le support, et compte tenu du réglage du positionnement et de l'enfoncement du bâton dans le support.

Un procédé de fabrication d'une composition cosmétique est décrit dans le document DE 33 27 771 A1, ou dans le document US 5 160 689 A, par exemple.

Un but de l'invention est donc d'obtenir un procédé plus productif de réalisation d'une composition cosmétique structurée par moulage, sans nuire à la qualité, notamment esthétique, de la composition obtenue.

A cet effet, l'invention a pour objet un procédé selon la revendication 1.

Le procédé selon l'invention peut comprendre l'une ou plusieurs des caractéristiques des revendications 2 à 11.

L'invention sera mieux comprise à la lecture de la description qui va suivre, donnée uniquement à titre d'exemple, et faite en se référant aux dessins annexés, sur lesquels :
- la figure 1 est une vue schématique de côté d'un premier article cosmétique comprenant une composition cosmétique selon l'invention ;
- la figure 2 est une vue schématique, prise en coupe suivant un plan axial médian, d'une installation de fabrication de l'article cosmétique selon l'invention, lors d'une phase d'injection de la composition cosmétique ;
- la figure 3 est une vue analogue à la figure 2, lors d'une phase préalable d'injection du support de la composition cosmétique.

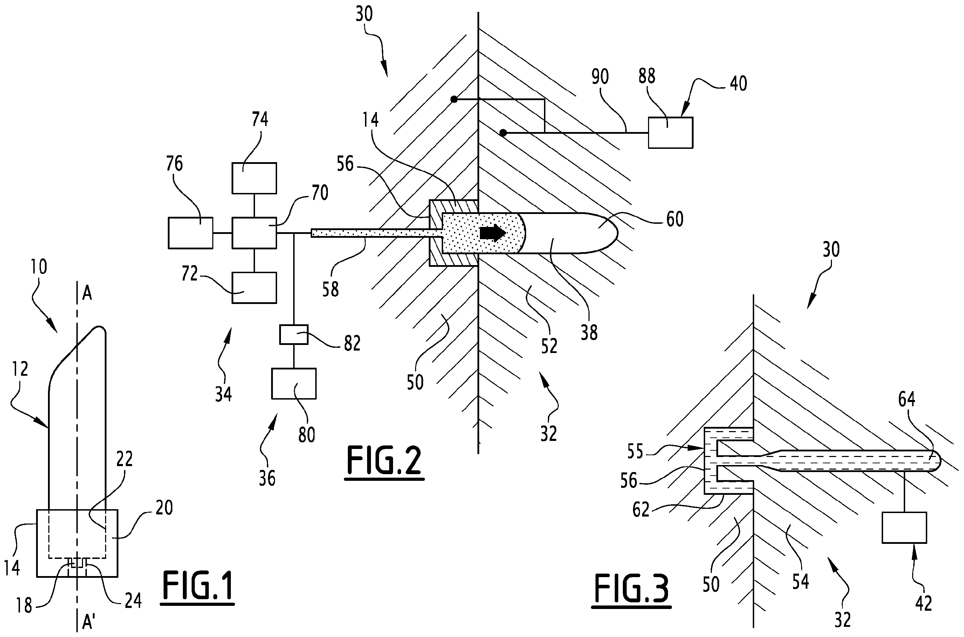

Un premier article cosmétique 10 réalisé par un procédé selon l'invention est illustré par la figure 1. L'article cosmétique 10 comporte une composition cosmétique 12, et avantageusement, un support 14 destiné à porter la composition cosmétique 12.

La composition cosmétique 12 comporte au moins un produit cosmétique. Elle forme par exemple un rouge à lèvres, un stick, une poudre hybride, un produit déodorant et/ou anti-transpirant, un savon, un masque visage, une cire de coiffage, ou/et un parfum solide.

A température ambiante, par exemple à 25 °C, la composition cosmétique 12 est structurée. Par « structurée », on entend notamment au sens de la présente invention que la composition cosmétique 12 présente une tenue mécanique propre, c'est-à-dire qu'elle conserve spontanément sa forme, durant toute la durée de vie du produit en l'absence de sollicitation extérieure. Cette durée de vie est par exemple d'au moins un jour, et notamment d'au moins un an.

Ainsi, la composition cosmétique 12 dans l'article cosmétique 14 formé à l'issue du procédé selon l'invention n'est pas liquide à température ambiante, et ne s'écoule pas spontanément de manière visible macroscopiquement pendant au moins une minute et avantageusement, pendant au moins un jour, en l'absence de sollicitation extérieure.

De préférence, la composition cosmétique 12 est solide. Dans ce cas, elle peut être saisie et déplacée par l'utilisateur, sans s'écouler.

La composition cosmétique 12 est applicable sur une surface corporelle de l'utilisateur, c'est-à-dire qu'elle est propre à détacher une partie d'elle-même pour rester sur la surface corporelle.

Avantageusement, la composition cosmétique 12 est applicable par frottement contre la surface corporelle de l'utilisateur, sans détérioration physique de la surface corporelle. En variante, la composition cosmétique 12 est applicable par frottement sur un applicateur.

Dans cet exemple, la composition cosmétique 12 comporte un bloc 16 solide formé de matériau cosmétique, et une saillie d'injection 18, également formée de matériau cosmétique, faisant saillie par rapport au bloc 16.

La composition cosmétique 12 est formée d'au moins un matériau cosmétique. Le ou chaque matériau cosmétique comporte au moins un composé fusible et avantageusement un composé additionnel.

Le ou chaque composé fusible est propre à être chauffé pour fondre et se fluidifier en vue de l'injection du matériau cosmétique. Il est propre à refroidir pour se solidifier en adoptant une forme imposée par la cavité de moulage.

Le ou chaque composé fusible est avantageusement un agent structurant. On entend par agent structurant un composé apte à augmenter la viscosité de la composition l'incorporant. L'agent structurant permet notamment d'obtenir une composition pouvant présenter une texture allant des textures fluides à solides.

Le ou chaque composé fusible est par exemple choisi parmi les cires, les argiles organophiles, les silices pyrogénées, les acides gras, les composés pâteux, les agents gélifiants, notamment les organogélateurs, les agents épaississants, les résines glutamides, les celluloses hydrophobes, les résines tackifiantes, et leurs mélanges.

Dans l'exemple représenté sur la figure 1, la composition cosmétique 12 comporte au moins un composé fusible formé d'une cire. La teneur massique en cire dans la composition cosmétique 12 est par exemple supérieure à 10 %.

Le ou chaque composé additionnel est choisi par exemple parmi les huiles, les pâtes, les pigments, les nacres, les charges, les tensioactifs, les séquestrants, les neutralisants, les antioxydants, les colorants, les parfums, les actifs cosmétiques, les réducteurs.

Dans l'exemple représenté sur la figure 1, le bloc 16 fait saillie le long d'un axe A-A' par rapport au support 14. Il est reçu partiellement dans le support 14. La saillie d'injection 18 est masquée par le support 14. Elle présente une hauteur, prise le long de l'axe A-A' très inférieure à la hauteur du bloc 16.

Le bloc 16 forme ici un raisin de produit.

Le matériau de support solide peut être un thermoplastique, par exemple choisi parmi le polyoxyméthylène (POM), le polyvinyl toluène (PVT), ou le polycyclohexanediméthanol acide téréphtalique (PCTA).

Le support 14 comprend ici une paroi creuse 20 qui délimite une cavité 22 de réception de la composition cosmétique 12. Le fond de la paroi 20 délimite avantageusement un passage 24 d'injection de la composition cosmétique 12 débouchant dans la cavité 22. La saillie d'injection 18 est de préférence logée dans le passage 24.

Dans cet exemple, le bloc 16 remplit totalement la cavité 22. Il s'applique sur toute la surface de la paroi 20 délimitant la cavité 22. Le bloc 16 est ainsi fixé dans la cavité 22.

En variante, la paroi 20 du support 14 n'est pas creuse. La composition cosmétique 12 est simplement placée au contact de la paroi 20.

L'article cosmétique 10 est fabriqué par un procédé selon l'invention dans une installation 30 représentée schématiquement sur les figures 2 et 3.

L'installation 30 comporte un moule d'injection 32, un étage 34 d'injection dans le moule 32 du matériau cosmétique destiné à former la composition cosmétique 12, et, selon l'invention, un ensemble 36 de mise sous vide d'une cavité de moulage 38 du moule d'injection 32.

L'installation 30 comporte avantageusement un ensemble de refroidissement 40 de la cavité de moulage 38.

Dans l'exemple représenté sur la figure 3, l'installation 30 comporte en outre un étage d'injection 42 dans le moule 32 d'un matériau de support destiné à former le support 14.

Comme illustré par la figure 2, le moule 32 comporte un demi-moule 50 de base, un demi-moule 52 de formation de la composition cosmétique 12, visible sur la figure 2, et avantageusement, un demi-moule 54 de formation de support 14, visible sur la figure 3.

Dans cet exemple, le demi-moule de base 50 est mobile, par exemple en rotation, par rapport au demi-moule 52 et au demi-moule 54 pour former successivement une cavité de moulage 55 du support 14 et une cavité de moulage 38 de la composition cosmétique 12.

Le demi-moule de base 50 définit une empreinte 56 de formation d'une partie de la composition cosmétique 12, et de réception du support 14. Il délimite un canal 58 d'amenée du matériau cosmétique fluide destiné à former la composition cosmétique 12.

L'empreinte de formation 56 débouche à l'extérieur du demi-moule 50. Elle est destinée à être positionnée en regard de chaque demi-moule 52, 54 lors de l'injection pour former une partie de la cavité de moulage respective 38, 55.

Le canal d'amenée 58 s'étend à travers le demi-moule 50. Il débouche dans le fond de l'empreinte 56.

En référence à la figure 2, le demi-moule 52 de formation de la composition cosmétique 12 délimite une empreinte 60 de formation d'une deuxième partie de la composition cosmétique 12.

Le demi-moule 52 est propre à se plaquer sur le demi-moule de base 50 pour placer l'empreinte 56 en regard de l'empreinte 60 et délimiter la cavité de moulage 38, de forme complémentaire à la composition cosmétique 12.

En référence à la figure 3, le demi-moule 54 de formation du support 12 délimite une saillie 62 destinée à s'insérer dans l'empreinte 56. Le demi-moule 54 définit un canal 64 d'amenée de matériau de support fluide, débouchant ici dans la saillie 62.

Le demi-moule 54 est propre à se plaquer sur le demi-moule de base 50 pour placer la saillie 62 dans l'empreinte 56 et délimiter la cavité de moulage 55, de forme complémentaire au support 14.

Comme visible sur la figure 2, l'étage d'injection 34 comporte un réservoir 70 (ou « fondoir »), recevant le matériau cosmétique fondu destiné à former la composition cosmétique 12, un élément 72 de chauffage du réservoir 70, un ensemble 74 d'élimination des bulles dans le matériau cosmétique fondu, et un ensemble 76 de mise en pression du matériau cosmétique fondu pour le pousser vers la cavité de moulage 38 et pour le maintenir en pression dans la cavité de moulage 38.

L'élément de chauffage 72 est propre à piloter la température dans le réservoir 70 pour chauffer et maintenir en température le matériau présent dans le réservoir 70 à une température de commande, avantageusement comprise entre 50° C et 120° C.

L'ensemble d'élimination des bulles 74 comporte au moins une source de mise en dépression par exemple une pompe à vide.

L'ensemble de mise en pression 76 comporte un système de mise sous pression mécanique, vis sans fin ou piston mécanique propre à mettre le produit liquide sous pression à une pression pilotée comprise avantageusement entre 0,2 bars relatifs et 2,5 bars relatifs.

L'ensemble de mise en dépression 36 comporte une source de vide, tel qu'une pompe à vide 80, et un contrôleur 82 propre à piloter la dépression à une valeur avantageusement comprise entre -1 bars relatif et -0,2 bars relatifs.

L'ensemble de mise en dépression 36 est ici raccordé à la cavité 38 par l'intermédiaire du canal d'amenée 58.

L'ensemble de refroidissement 40 comporte par exemple une source de réfrigération 88 et un circuit 90 de circulation d'un fluide caloporteur dans les parois du moule 32, avantageusement dans au moins un demi-moule 50, 52, 54.

L'ensemble de refroidissement 40 est ainsi propre à refroidir la surface du moule délimitant la cavité 38, à une température contrôlée comprise avantageusement entre 10°C et - 20°C, avantageusement inférieure à 5°C et autour de 0°C. Ces températures basses sont adaptées pour obtenir des caractéristiques spécifiques à l'état de surface ou sur des produits très mous.

En référence à la figure 3, l'étage d'injection 42 du matériau de support est propre à injecter le matériau de support sous forme fluide dans la cavité de moulage 55 à travers le canal d'amenée 64.

Un premier procédé de fabrication d'un article cosmétique 10 selon l'invention va maintenant être décrit, en regard des figures 2 et 3.

Initialement, le procédé comporte le remplissage du réservoir 70 avec du matériau cosmétique et la mise en chauffe du réservoir 70 par l'élément de chauffage 72 pour faire fondre le ou chaque composé fusible contenu dans le matériau cosmétique destiné à former la composition 12. La température du matériau cosmétique contenu dans le réservoir 70 est par exemple maintenue à une valeur de consigne comprise entre 50°C et 120°C.

L'ensemble 74 d'élimination des bulles est également activé pour dégazer le matériau cosmétique fluide.

Pour fabriquer chaque article cosmétique 10, le procédé comprend avantageusement une étape initiale de formation du support 14 par moulage.

Comme illustré par la figure 3, le demi-moule de base 50 est initialement placé au contact du demi-moule 54 de formation du support 14.

La saillie 62 du demi-moule 54 pénètre dans l'empreinte de base 56 pour former la cavité de moulage 55.

L'étage d'injection 42 de matériau de support est alors activé pour amener du matériau de support fluide dans la cavité de moulage 55 et la remplir.

Puis, un pion est introduit dans la cavité 55 pour aménager le passage traversant 24 dans le fond du support 14. En variante, le passage traversant 24 est formé par la géométrie de la cavité de moulage 55.

Le matériau de support fluide durcit par refroidissement dans la cavité 55 formant le support 14.

Puis, le demi-moule de base 50 est écarté du demi-moule 54 de formation du support 14 et est appliqué contre le demi-moule 52 de formation de la composition cosmétique 12.

L'empreinte 60 se place en regard de l'empreinte de base 56, pour former la cavité de moulage 38 qui contient initialement le support 14 fabriqué à l'étape précédente.

L'ensemble de mise en dépression 36 est alors activé. La dépression dans la cavité de moulage 38 est alors réglée et maintenue à une valeur comprise par exemple entre -1 bars relatif et -0,2 bars relatifs.

Ensuite, la cavité 38 étant en dépression, l'ensemble de mise en pression 76 est activé pour pousser le matériau cosmétique fluide à travers le canal d'amenée 58, puis à travers le passage 24 dans la cavité de moulage 68. La pression d'injection est par exemple comprise entre 0,2 bars relatifs et 2,5 bars relatifs.

Simultanément, l'ensemble de refroidissement 40 est activé pour maintenir la surface du moule 32 délimitant la cavité 38 à une température de refroidissement prédéfinie, avantageusement comprise entre -20°C et 10°C, notamment entre 10°C et - 1°C. Cette température de refroidissement est très basse pour favoriser un certain type de cristallisation (choc thermique).

Le composé fusible présent dans le matériau cosmétique se solidifie par refroidissement du matériau cosmétique au contact de la surface du moule 32, et forme ainsi la composition cosmétique 12. Avantageusement, le refroidissement du matériau cosmétique est adapté pour atteindre par exemple la température ambiante (par exemple 25°C) et correspond à la différence entre la température d'injection et la température de sortie de moule. La pression est maintenue dans la cavité de moulage 38 par l'intermédiaire de l'ensemble de mise en pression 76 à une pression avantageusement comprise entre 0,2 bars relatifs et 2,5 bars relatifs, afin de compenser le retrait éventuel du matériau cosmétique lors de sa solidification.

Le moule 32 est ensuite ouvert pour éjecter l'article cosmétique 10 comprenant le support 14 et la composition cosmétique 12 portée par le support 14.

Le procédé selon l'invention augmente donc la cadence de production en diminuant le temps d'injection, compte tenu de la pression différentielle significative entre le matériau cosmétique fluide injecté sous pression et la pression régnant dans la cavité de moulage 38 après l'application d'une dépression.

La composition cosmétique formée est dépourvue de bulles d'air, puisque le matériau cosmétique injecté dans la cavité de moulage 38 a été préalablement dégazé et est injecté dans une atmosphère sous vide partiel, à l'aide d'un circuit fermé sous pression, et avec une vitesse de dosage très importante.

Par ailleurs, la cavité de moulage 38 étant maintenue en dépression, il est possible de descendre significativement la température des surfaces du moule 32 délimitant la cavité de moulage 38, sans risquer de condenser de la vapeur d'eau dans la cavité 38. Il est donc possible de refroidir le matériau cosmétique très rapidement, à l'aide d'un fluide caloporteur standard ou avec un pouvoir calorifique plus important, tel que l'azote liquide.

Par ailleurs, le support 14 étant présent initialement dans la cavité de moulage 38, la fixation de la composition cosmétique 12 sur le support 14 est parfaitement ajustée, puisque le matériau cosmétique remplit totalement la cavité 22 définie dans le support 14.

Les problèmes de tolérance de dimensions entre la composition cosmétique 12 et le support 14 des procédés de l'état de la technique sont résolus. De plus, la fixation de la composition cosmétique 12 s'effectuant directement lors de son injection, il n'est plus nécessaire de procéder à une étape de montage du support 14 sur la composition cosmétique 12 limitant les problèmes de positionnement ou d'enfoncement non conforme et/ou les problèmes de retrait du matériau formant la composition cosmétique 12.

L'état de surface de la composition cosmétique 12 et la précision de fabrication des formes et/ou décor présent sur cette surface est aussi amélioré grâce au maintien d'une pression dans le moule 32 à la fin de l'injection, conjuguée à une dépression dans la cavité 38 avant l'injection.

Dans une variante, non représentée, le support 14 est fabriqué préalablement dans une autre installation. L'installation 30 ne comporte alors pas de demi-moule 54 de formation du support 14.

Le procédé diffère alors de celui décrit précédemment en ce que le support 14 est introduit préalablement dans l'empreinte de base 56, avant la fermeture du moule 32 et avant la mise en dépression de la cavité de moulage 38.

Dans une autre variante, l'article cosmétique 10 est dépourvu de support 14. Il est alors constitué uniquement de composition cosmétique 12.

## Revendications

1. Procédé de fabrication d'une composition cosmétique (12) comprenant les étapes suivantes :
- chauffage d'au moins un matériau cosmétique contenant au moins un composé fusible pour faire fondre le ou chaque composé fusible,
- injection du matériau cosmétique chauffé dans une cavité de moulage (38) d'un moule (32) ;
- solidification au moins partielle du matériau cosmétique par refroidissement du matériau cosmétique à une température inférieure à celle du matériau cosmétique à l'étape de chauffage, pour former la composition cosmétique (12), la composition cosmétique (12) formée étant dépourvue de bulles d'air ;
- extraction de la composition cosmétique (12) hors du moule (32) ;
le procédé comprenant la mise en dépression de la cavité de moulage (38) du moule (32) avant et/ou pendant l'injection du matériau cosmétique chauffé.

2. Procédé selon la revendication 1, dans lequel la pression dans la cavité de moulage (38) est inférieure à -0,1 bars relatifs lors de la mise en dépression.

3. Procédé selon l'une quelconque des revendications précédentes, dans lequel le ou chaque matériau cosmétique chauffé est injecté sous pression dans la cavité de moulage (38), la pression d'injection du matériau cosmétique chauffé étant avantageusement comprise entre 0,2 bars relatifs et 2,5 bars relatifs.

4. Procédé selon l'une quelconque des revendications précédentes, dans lequel la cavité de moulage (38) est délimitée par une surface du moule (32), le procédé comprenant une étape de refroidissement de la surface du moule (32) avant et/ou pendant et/ou après l'étape d'injection.

5. Procédé selon la revendication 4, dans lequel la température de la paroi du moule (32) lors du refroidissement est inférieure à 5° C, notamment inférieure à 0°C.

6. Procédé selon l'une quelconque des revendications précédentes, comprenant, avant l'étape d'injection, la fabrication ou/et l'insertion dans la cavité de moulage (38) d'un support (14) solide, destiné à porter la composition cosmétique (12), le matériau cosmétique chauffé entrant en contact avec le support (14) lors de l'étape d'injection.

7. Procédé selon l'une quelconque des revendications précédentes, dans lequel dans lequel le ou chaque composé fusible est choisi parmi les cires, les argiles organophiles, les silices pyrogénées, les acides gras, les composés pâteux, les agents gélifiants, notamment les organogélateurs, les agents épaississants, les résines glutamides, les celluloses hydrophobes, les résines tackifiantes, et leurs mélanges.

8. Procédé selon la revendication 7, dans lequel la composition cosmétique (12) comporte une teneur massique en cire supérieure à 10 %.

9. Procédé selon l'une quelconque des revendications précédentes, dans lequel au moins un matériau cosmétique chauffé à l'étape de chauffage comporte en outre au moins un agent additionnel, notamment choisi parmi les huiles, les pâtes, les pigments, les nacres, les charges, les tensioactifs, les séquestrants, les neutralisants, les antioxydants, les colorants, les parfums, les actifs cosmétiques, les réducteurs.

10. Procédé selon l'une quelconque des revendications précédentes, dans lequel la composition cosmétique (12) est un rouge à lèvres, un stick, une poudre hybride, un produit déodorant et/ou anti-transpirant, un savon, un masque visage, une cire de coiffage, ou/et un parfum solide.

11. Procédé selon l'une quelconque des revendications précédentes, dans lequel, à l'étape de chauffage, le matériau cosmétique est chauffé à une température entre 50°C et 120°C.

## Patentansprüche

1. Herstellungsverfahren einer kosmetischen Zusammensetzung (12), umfassend die folgenden Schritte:
- Erwärmen mindestens eines kosmetischen Materials, das mindestens eine schmelzbare Verbindung enthält, um die oder jede schmelzbare Verbindung zu schmelzen,
- Einspritzen des erhitzten kosmetischen Materials in einen Formhohlraum (38) einer Form (32);
- mindestens teilweises Verfestigen des kosmetischen Materials durch Abkühlen des kosmetischen Materials auf eine Temperatur, die niedriger ist als jene des kosmetischen Materials in dem Erwärmungsschritt, um die kosmetische Zusammensetzung (12) zu bilden, wobei die gebildete kosmetische Zusammensetzung (12) frei von Luftblasen ist;
- Entnehmen der kosmetischen Zusammensetzung (12) aus der Form (32);
wobei das Verfahren das Unterdrucksetzen des Formhohlraums (38) der Form (32) vor und/oder während des Einspritzens des erwärmten kosmetischen Materials umfasst.

2. Verfahren nach Anspruch 1, wobei der Druck in dem Formhohlraum (38) bei dem Unterdrucksetzen weniger als relative -0,1 bar ist.

3. Verfahren nach einem der vorherigen Ansprüche, wobei das oder jedes erwärmte kosmetische Material unter Druck in den Formhohlraum (38) eingespritzt wird, wobei der Einspritzdruck des erwärmten kosmetischen Materials vorteilhafterweise zwischen 0,2 relativen bar und 2,5 relativen bar ist.

4. Verfahren nach einem der vorherigen Ansprüche, wobei der Formhohlraum (38) durch eine Oberfläche der Form (32) begrenzt ist, wobei das Verfahren einen Schritt eines Kühlens der Oberfläche der Form (32) vor und/oder während und/oder nach dem Einspritzschritt umfasst.

5. Verfahren nach Anspruch 4, wobei die Temperatur der Formwand (32) beim Abkühlen weniger als 5 °C, insbesondere weniger als 0 °C, ist.

6. Verfahren nach einem der vorherigen Ansprüche, umfassend vor dem Injektionsschritt die Herstellung oder/und das Einsetzen eines festen Trägers (14) in den Hohlraum des Formhohlraums (38), der dazu bestimmt ist, die kosmetische Zusammensetzung (12) zu tragen, wobei das erwärmte kosmetische Material während des Einspritzschritts mit dem Träger (14) in Kontakt kommt.

7. Verfahren nach einem der vorherigen Ansprüche, wobei die oder jede schmelzbare Verbindung ausgewählt ist aus Wachsen, organophilen Tonen, pyrogenen Kieselsäuren, Fettsäuren, pastösen Verbindungen, Geliermitteln, insbesondere Organogeliermittel, Verdickungsmitteln, Glutamidharzen, hydrophoben Cellulosen, klebrigmachenden Harzen und deren Gemischen.

8. Verfahren nach Anspruch 7, wobei die kosmetische Zusammensetzung (12) einen Massengehalt an Wachs von mehr als 10 % umfasst.

9. Verfahren nach einem der vorherigen Ansprüche, wobei mindestens ein kosmetisches Material, das in dem Erwärmungsschritt erwärmt wird, ferner mindestens einen zusätzlichen Wirkstoff umfasst, der insbesondere ausgewählt aus Ölen, Pasten, Pigmenten, Perlmutt, Füllstoffen, Tensiden, Sequestriermitteln, Neutralisatoren, Antioxidantien, Farbstoffen, Parfüms, kosmetischen Wirkstoffen, Reduktionsmitteln.

10. Verfahren nach einem der vorherigen Ansprüche, wobei die kosmetische Zusammensetzung (12) ein Lippenstift, ein Stick, ein Hybridpuder, ein Deodorant und/oder Antitranspirant, eine Seife, eine Gesichtsmaske, ein Stylingwachs oder/und ein festes Parfum ist.

11. Verfahren nach einem der vorherigen Ansprüche, wobei das kosmetische Material in dem Erwärmungsschritt auf eine Temperatur zwischen 50 °C und 120 °C erwärmt wird.

## Claims

1. Method of manufacturing a cosmetic composition (12) comprising the following steps:
- heating at least one cosmetic material containing at least one fuse compound to melt the or each fuse compound,
- injection of the heated cosmetic material into a moulding cavity (38) of a mould (32);
- at least partial solidification of the cosmetic material by cooling the cosmetic material to a temperature lower than that of the cosmetic material in the heating step, to form the cosmetic composition, to form the cosmetic composition (12), the cosmetic composition (12) formed being free of air bubbles;
- extraction of the cosmetic composition (12) from the mould (32);
the method comprising evacuating the mould cavity (38) of the mould (32) before and/or during injection of the heated cosmetic material.

2. Method according to claim 1, in which the pressure in the moulding cavity (38) is less than -0.1 relative bar when the vacuum is applied.

3. Method according to any one of the preceding claims, in which the or each heated cosmetic material is injected under pressure into the moulding cavity (38), the injection pressure of the heated cosmetic material advantageously being between 0.2 relative bars and 2.5 relative bars.

4. Method according to any one of the preceding claims, in which the moulding cavity (38) is limited by a surface of the mould (32), the method comprising a step of cooling the surface of the mould (32) before and/or during and/or after the injection step.

5. Method according to claim 4, in which the temperature of the mould wall (32) during cooling is less than 5 °C, in particular less than 0 °C.

6. Method according to any one of the preceding claims, comprising, prior to the injection step, the manufacture and/or insertion into the moulding cavity (38) of a solid support (14) intended to carry the cosmetic composition (12), the heated cosmetic material coming into contact with the support (14) during the injection step.

7. Method according to any one of the preceding claims, in which the or each fusible compound is chosen from waxes, organophilic clays, fumed silicas, fatty acids, pasty compounds, gelling agents, in particular organogelling agents, thickening agents, glutamide resins, hydrophobic celluloses, tackifying resins, and mixtures thereof.

8. Method according to claim 7, in which the cosmetic composition (12) comprises a wax content by weight over 10%.

9. Method according to any one of the preceding claims, in which at least one cosmetic material heated in the heating step additionally comprises at least one additional agent, chosen in particular from oils, pastes, pigments, pearlescents, fillers, surfactants, sequestrants, neutralisers, antioxidants, colourants, perfumes, cosmetic active ingredients and reducers.

10. Method according to any one of the preceding claims, in which the cosmetic composition (12) is a lipstick, a stick, a hybrid powder, a deodorant and/or antiperspirant product, a soap, a face mask, a styling wax, and/or a solid perfume.

11. Method according to any one of the preceding claims, in which, in the heating step, the cosmetic material is heated to a temperature of between 50 °C and 120 °C.
